# EUROPEAN PATENT APPLICATION

(11) **EP 3 170 530 A2**
(43) Date of publication of application: **24.05.2017**
(21) Application number: 16199467.8
(22) Date of filing: 18.11.2016
(51) Int. Cl.: A61N 5/06, A61N 7/00, A61N 1/40, A61B 18/18, A61B 18/20

(54) **CIRCUMFERENTIAL NECK TONING METHOD**

(30) Priority: 18.11.2015 US 201514945164
(71) Applicant: Reil, Julie Ann, Billings, MT 59102 (US)
(72) Inventor: Reil, Julie Ann, Billings, MT 59102 (US)
(74) Representative: Nguyen Van Yen, Christian

(57) **Abstract**

A method of toning skin on a human patient's neck. The method includes applying a beam to an exterior surface of a portion of the skin of the neck. The beam comprises at least one of infrared light waves, ultrasound waves, and low fluence radiofrequency waves. The beam is configured to tone the portion of the skin. The portion may include a posterior portion of the skin of the neck. In such embodiments, the beam tones the posterior portion causing the posterior portion to pull on and tighten an anterior portion of the skin. The portion of the skin may include a circumferential portion that extends circumferentially completely around the neck. The skin is divisible circumferentially around the neck into a plurality of sections that each extend only partway around the neck. The beam may be applied to each of the plurality of sections separately.

## Description

### BACKGROUND OF THE INVENTION

### Field of the Invention

The present invention is directed generally to methods of improving the appearance of the human neck by toning or tightening the skin and/or related tissue.

### Description of the Related Art

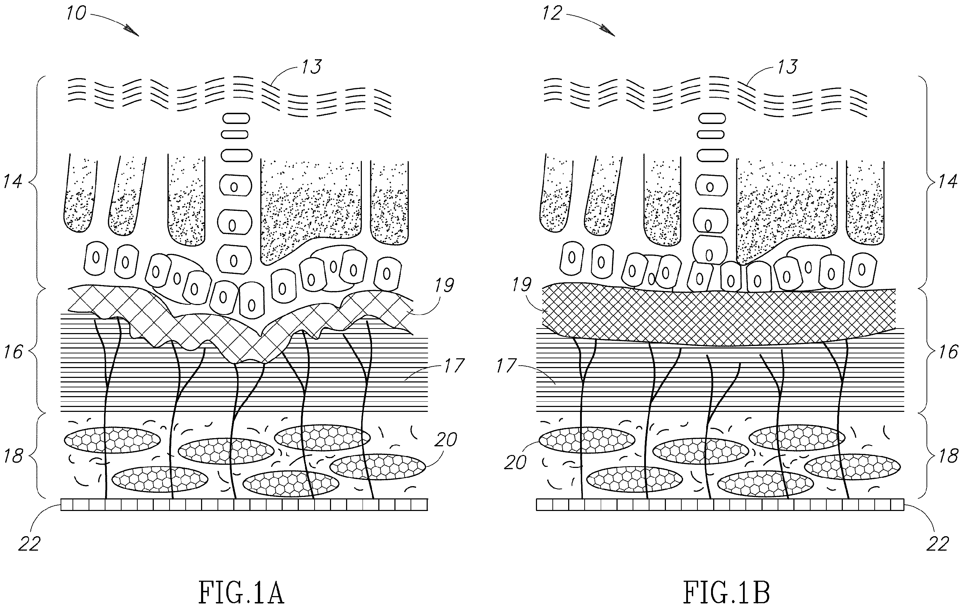

Figure 1 A is an illustration of a cross-section of old skin 10, and Figure 1 B is an illustration of a cross-section of young skin 12. Both the old skin 10 and the young skin 12 have the same anatomical structures. For example, the old skin 10 and the young skin 12 each have an epidermis 14, a dermis 16, and a hypodermis 18. Both the old skin 10 and the young skin 12 have an exterior surface 13 that is an outermost portion of the epidermis 14. A lower layer of the dermis 16 is referred to as a reticular dermis 17, and a layer above the reticular dermis 17 is referred to as a papillary dermis 19. Connective tissue 20 lies within the hypodermis 18, and fascia 22 is under or at the base of the hypodermis 18. The fascia 22 surrounds muscles, ligaments, and tendons and anchors the skin to bony prominences.

Collagen is abundant in the reticular dermis 17, and elastin is found mainly in the papillary dermis 19. Collagen resists stretching and provides structure to the skin. Elastin gives skin resiliency and the ability to stretch and snap back into shape (like a rubber band). Unfortunately, collagen and elastin levels in the structures of the young skin 12 decrease with aging and photodamage. As the volume of these compounds is depleted, the skin becomes more lax, sags, and wrinkles. Thus, as illustrated in Figures 1 A and 1 B, the old skin 10 is more lax and less toned than the young skin 12.

Many people are dissatisfied with the appearance of their neck. For example, many people believe the skin on their neck has wrinkled and/or sagged in an undesirable or unattractive manner. To address this problem, a number of surgical procedures have been developed to improve the appearance of the neck. For example, a conventional neck lift or platysmaplasty removes some skin from the neck, and repositions and/or tightens other skin. Unfortunately, such surgical interventions are painful, require recovery time, and may have undesirable complications (e.g., infections, scarring, nerve damage, tissue damage, etc.). Further, some people are simply not good surgical candidates and cannot take advantage of such procedures. Therefore, a need exists for methods of improving the appearance of the neck. Methods that do not involve surgery are particularly desirable. The present application provides these and other advantages as will be apparent from the following detailed description and accompanying figures.

### BRIEF DESCRIPTION OF THE SEVERAL VIEWS OF THE DRAWING(S)

Figure 1 A is an illustration of a cross-section of old skin.
Figure 1 B is an illustration of a cross-section of young skin.
Figure 2 is an illustration of a patient having the posterior portion of her neck toned by a beam emitting device operated by a healthcare provider.
Figure 3 is a cross-section of a portion of the beam emitting device illustrated treating a portion the patient's skin.
Figure 4 is an illustration of a posterior neck toning treatment grid.
Figure 5 is an illustration of a right posterior-lateral neck toning treatment grid.
Figure 6 is an illustration of a right lateral neck toning treatment grid.
Figure 7 is an illustration of a right anterior-lateral neck toning treatment grid.
Figure 8 is an illustration of an anterior neck toning treatment grid.
Figure 9 is an illustration of a left anterior-lateral neck toning treatment grid.
Figure 10 is an illustration of a left lateral neck toning treatment grid.
Figure 11 is an illustration of a left posterior-lateral neck toning treatment grid.
Figure 12 is an illustration of the healthcare provider using the beam emitting device to apply a beam to the anterior neck toning treatment grid.
Figure 13 is an illustration of the healthcare provider applying gel to the anterior neck toning treatment grid.
Figure 14 is an illustration of the healthcare provider using the beam emitting device to apply the beam to the anterior neck toning treatment grid through the gel.
Figure 15A is a photograph depicting an anterior portion of a patient's neck before treatment.
Figure 15B is a photograph depicting the anterior portion of the same patient's neck after treatment.

### DETAILED DESCRIPTION OF THE INVENTION

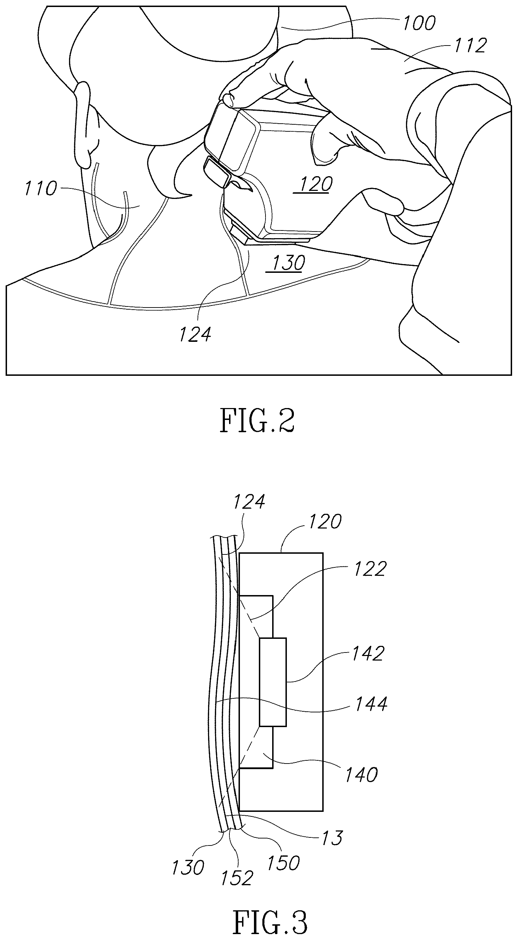

Figure 2 depicts a patient 100 with a neck 110. A healthcare provider 112 (e.g., a physician, nurse, technician, and the like) operates a beam emitting device 120 and positions it to deliver a beam 122 (e.g., light, ultrasound, or radiofrequency) to at least a portion of a treatment area 124. The treatment area 124 may include the entire neck 110 or a portion thereof. For example, the treatment area 124 may include a circumferentially extending portion of the neck 110. The treatment area 124 may also include portions of the shoulders, lower jaw (e.g., under the chin), upper chest, and/or upper back of the patient.

The beam 122 is applied to the exterior surface 13 (see Figures 1 A and 1B) of skin 130 in the treatment area 124. Referring to Figures 1 A and 1B, like the old skin 10 and the young skin 12, the skin 130 (see Figure 2) includes the epidermis 14, the dermis 16, and the hypodermis 18. Further, the connective tissue 20 lies within the hypodermis 18, and the fascia 22 is located under (or at the base of) the hypodermis 18. In particular, the beam 122 may be delivered to structures composed of various subtypes of collagen (types I-XII) and subtypes of elastin (I-VI) depending on depth and function. For example, the beam 122 may be delivered to the dermis 14, the connective tissue 20, and the fascia 22 within the treatment area 124.

As illustrated in Figure 3, the device 120 may include a treatment window 140 through which the beam 122 is emitted from a source 142. The treatment window 140 may be configured to be placed near or against the exterior surface 13 of the skin 130 in at least a portion of the treatment area 124. If the treatment window 140 is smaller than the treatment area 124, the beam 122 will be applied to only a portion of the treatment area 124. For ease of illustration, the beam 122 will be described as being applied to a target region 144. As is apparent to those of ordinary skill in the art, the treatment area 124 may include one or more target regions. If necessary to treat the entire treatment area 124, the treatment window 140 may be repositioned within the treatment area 124 to apply the beam 122 on different target regions until the entire treatment area 124 has been treated. The different target regions may be non-overlapping, or partially overlapping. By way of a non-limiting example, the beam 122 may be applied to one or more target regions located circumferentially around the neck 110 by positioning and/or repositioning the treatment window 140 to apply the beam 122 to those target regions within the treatment area 124.

The beam 122 may be delivered in a continuous pulsed delivery or intermittent pulsed delivery. The beam 122 may include light, ultrasound, and/or low fluence radiofrequency ("RF"). Low fluence RF may be delivered at a low level that does not cause pain and at the same time penetrates the skin to a satisfactory depth (e.g., up to about 20 mm). In contrast, higher fluence radiofrequency treatments can penetrate beyond 20 mm into the skin and deliver more heat and are frequently described by patients as being painful. By way of another non-limiting example, the source 142 may be implemented using any source suitable for delivering low fluence RF (e.g., suitable devices may be purchased from Cynosure, Inc., which acquired Palomar Medical Technologies, Inc., Alma Lasers, Ltd., and the like). Non-limiting examples of suitable devices include the Pellevé S5 RF System manufactured by Cynosure, Inc., the Accent Family of RF based platforms manufactured by Alma Lasers, Ltd., and the like.

In embodiments in which the beam 122 delivers ultrasound waves, the source 142 may be implemented using a micro pinpoint ultrasound device. By way of another non-limiting example, the source 142 may be implementing using any source suitable for delivering ultherapy.

In embodiments in which the beam 122 delivers light, the light may be near infrared light (700 nanometers ("nm") - 1200 nm), mid infrared light (900 nm - 1400 nm) or far infrared light (1300 nm - 1800 nm), or broadband light (e.g., having wavelengths of approximately 695 nm to greater than approximately 800 nm). By way of a non-limiting example, the light may include only infrared light. By way of another non-limiting example, the light may have wavelengths of about 700 nm (near infrared) to about 1,800 nm (far infrared). The source 142 may be implemented using an infrared light source, a broadband spectrum light source, and the like.

The beam 122 may penetrate a predetermined distance into the target region 144. For example, the beam 122 may penetrate into or through the skin 130 only about 1 mm to about 3 mm. In contrast, radiofrequency treatments can penetrate beyond 20 mm in the skin and are frequently described by patients as being painful.

While the beam 122 may include light, the beam 122 is not a laser of the type commonly used for facial resurfacing. Collagen fibers are abundant in the reticular dermis 17, which is a lower layer of the dermis 16. On the other hand, the epidermis 14 (see Figures 1A and 1B), which is above the dermis 16, is nearly devoid of collagen fibers. A laser of the type used for facial resurfacing cannot reach the reticular dermis 17 (to shrink collagen therein) without damaging or denaturing collagen.

The source 142 may be capable of controlling skin temperature. Collagen fibers shrink when heated. Thermal shrinkage of collagen occurs between about 55°C and about 58°C. Thus, the source 142 may be used to heat the skin 130 to between about 55°C and about 58°C to help tone the skin 130. Treatments utilizing the source 142 may not be painful or require anesthesia.

Without being limited by theory, it is believed that the beam 122 (e.g., infrared light, ultrasound, or broad band light) may tone skin through a process referred to as biostimulation, which is defined as the use of light or ultrasound to direct the body to repair damaged skin and tissue components. Fibroblasts produce collagen and elastin, which are structural components of skin, muscle, and connective tissue. Fibroblast cells can repair elastin and collagen in skin, muscle, ligaments, and tendons. When skin is lacerated, fibroblasts repair the damage by making a scar. Fibroblasts can also be stimulated by the beam 122 to repair damaged skin and tissue in the absence of a laceration or wound. For example, when stimulated by the beam 122, fibroblasts may repair damaged collagen fibers in the reticular dermis and/or synthesize new collagen fibers (neocollagenesis). Collagen fibers give structural support to skin. When stimulated by the beam 122, fibroblasts may repair damaged elastin fibers in the papillary dermis and/or synthesize new elastin fibers (neoelastigenesis). Elastin fibers give stretchability to the skin. Thus, referring to Figure 2, the beam 122 may be used to help tone the skin 130 (and improve the appearance the skin 130) by stimulating fibroblasts to repair and/or synthesize collagen fibers and/or elastin fibers.

Referring to Figure 2, by way of non-limiting examples, the beam emitting device 120 may be implemented as a deep dermal heating device, such as a TITAN device, which can deliver light having a wavelength within a range of about 1100 nm -to about 1800 nm (available from Cutera, Inc.), or a broadband light device, such as a BBL^{™} SkinTyte ("ST") BroadBand Light device, which can deliver light having wavelengths of about 700 nm to about 1300 nm) (available from Sciton, Inc). Other non-limiting examples of devices that may be used to implement the beam emitting device 120 are described in U.S. patent application publication No. 2005/0049658, titled "Method and System for Treatment of PostPartum Abdominal Skin Redundancy or Laxity," and U.S. patent application publication No. 2006/0052847, titled "System and Method for Heating Skin Using Light to Provide Tissue Treatment."

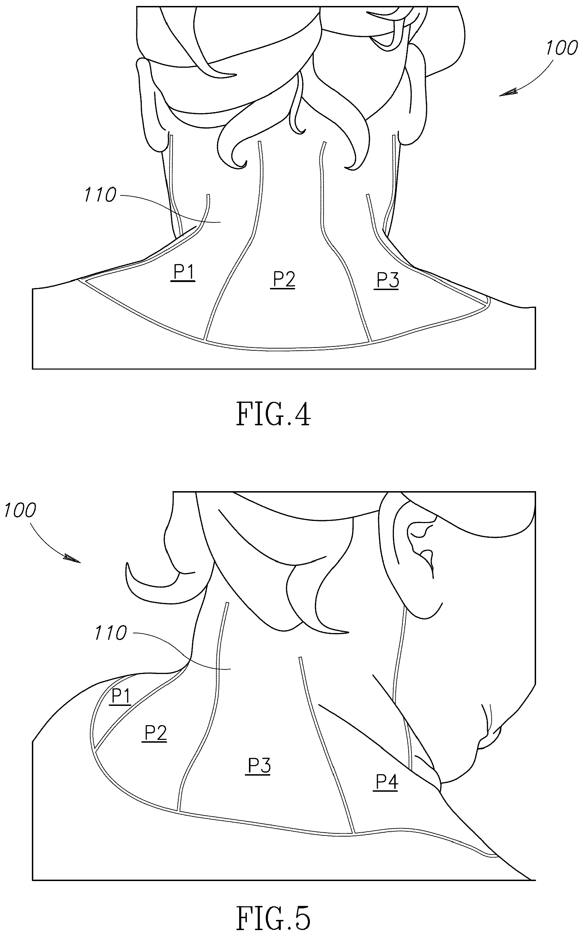

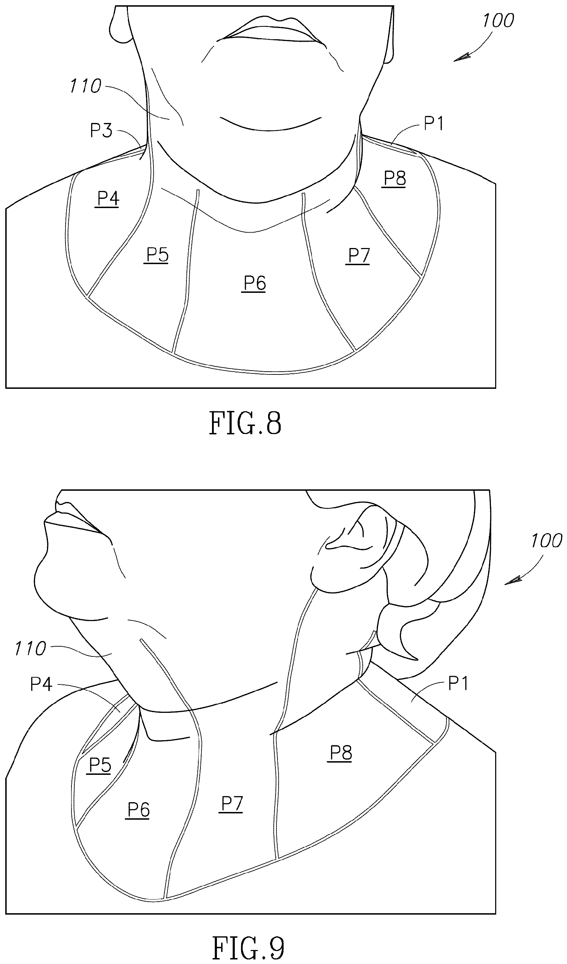

As explained above, the beam 122 may be applied to the skin 130 and used to improve both its structure (by repairing and making new collagen fibers) and its elasticity (by repairing and making new elastin fibers). Thus, if the beam 122 is used to treat a front portion of the neck 110, one would expect the structure and elasticity of that portion to improve. The inventor found that using the beam 122 to treat the back (or posterior) of the neck 110 (see Figures 2 and 4) may also improve the appearance of the front (or anterior) of the neck 110 (see Figures 8, 12-14, and 15A-15B). In particular, treating the back of the neck 110 was found to improve the appearance of the front of the neck 110 in thin, athletic women. In these patients, about 50% to about 75% (and sometimes even 100%) of the forward sagging skin and soft tissue on the front of the neck 110 was improved after the beam 122 was used to tone the back of the neck. Essentially, the skin 130 on the rear of the neck 110 was tightened (or pulled taut), which pulled the skin on other parts of the neck rearwardly and immediately improved the appearance of the front of the neck.

The back of the neck 110 is very delicate because it contains nerves, arteries, and muscles that are so close to the surface that it is impossible to dissect out those structures and perform a surgical "lift" or toning. The inventor determined that the back of the neck 110 may be treated safely with the beam 122. Thus, the beam 122 may be used to treat portions of the neck 110 that may not be treated surgically.

Referring to Figures 4-11, the neck 110 may be divided circumferentially into eight grids each including three sections. When one of the grids is treated, the beam 122 (see Figure 3) is applied to each of the sections in the grid. For example, Figure 4 depicts sections P1-P3 defined on the back or posterior of the neck 110. Together, the sections P1-P3 form a posterior neck toning treatment grid. In Figure 2, the healthcare provider 112 is using the device 120 to treat the posterior neck toning treatment grid. Returning to Figure 4, when treating the posterior neck toning treatment grid, the beam 122 (see Figure 3) is applied to each of the sections P1-P3.

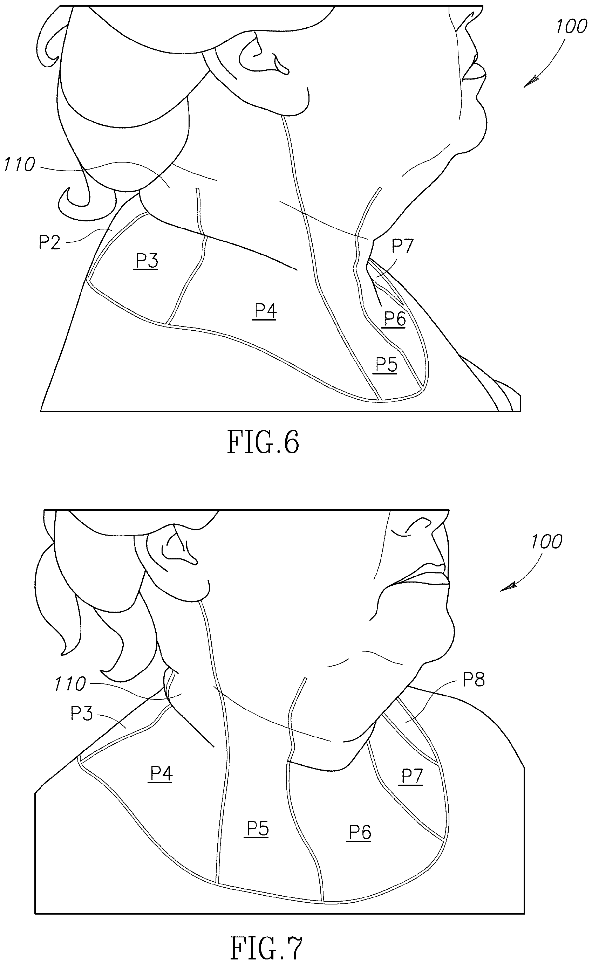

Figure 5 depicts sections P2-P4 defined on right posterior-lateral portion of the neck 110. Together, the sections P2-P4 form a right posterior-lateral neck toning treatment grid. Figure 6 depicts sections P3-P5 defined on right lateral portion of the neck 110. Together, the sections P3-P5 form a right lateral neck toning treatment grid. Figure 7 depicts sections P4-P6 defined on right anterior-lateral portion of the neck 110. Together, the sections P4-P6 form a right anterior-lateral neck toning treatment grid.

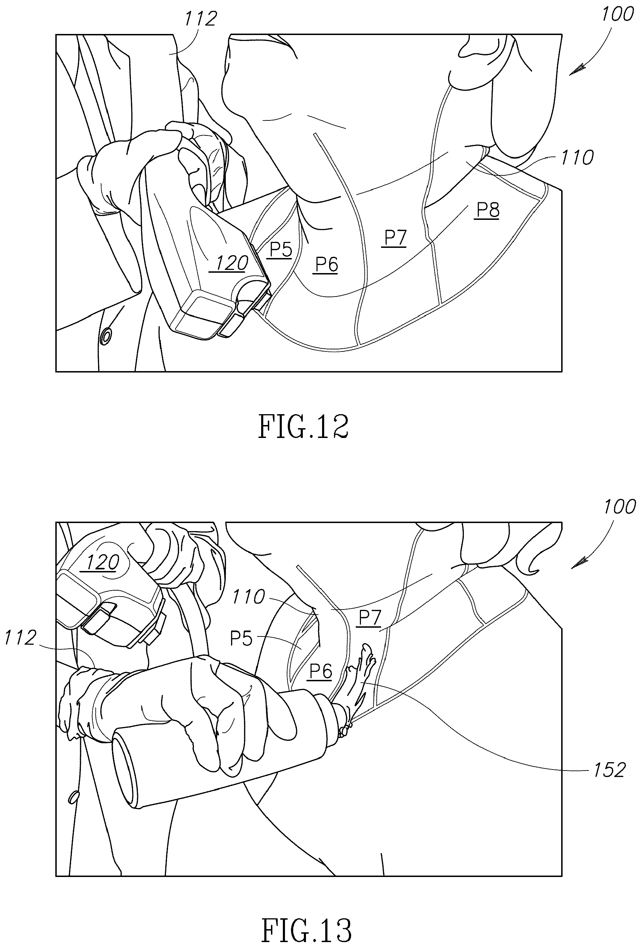

Figure 8 depicts sections P5-P7 defined on an anterior portion of the neck 110. Together, the sections P5-P7 form an anterior neck toning treatment grid. In Figure 12, the healthcare provider 112 is using the device 120 to treat the anterior neck toning treatment grid. Returning to Figure 8, when treating the anterior neck toning treatment grid, the beam 122 (see Figure 3) is applied to each of the sections P5-P7.

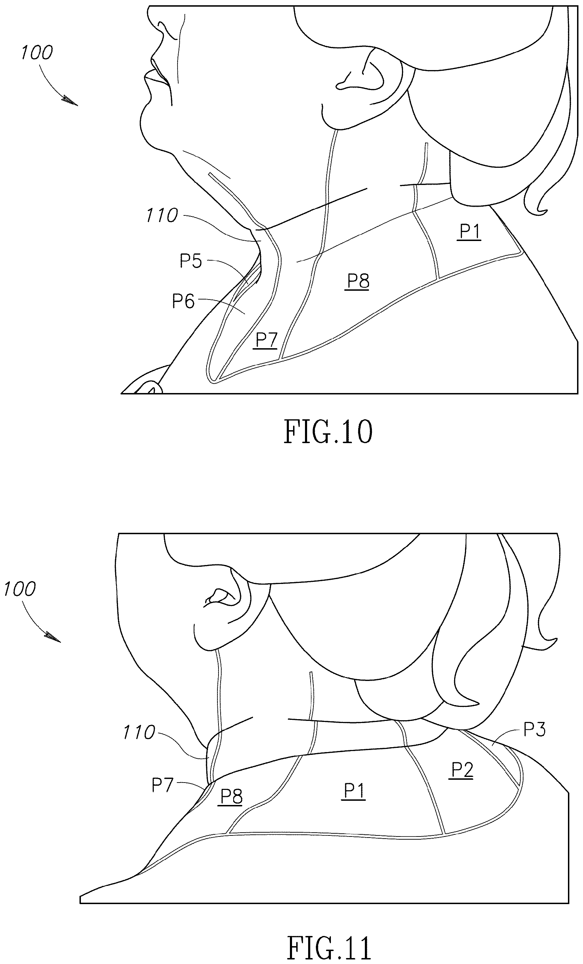

Figure 9 depicts sections P6-P8 defined on a left anterior-lateral portion of the neck 110. Together, the sections P6-P8 form a left anterior-lateral neck toning treatment grid. Figure 10 depicts sections P7, P8, and P1 defined on left lateral portion of the neck 110. Together, the sections P7, P8, and P1 form a left lateral neck toning treatment grid. Figure 11 depicts sections P8, P1, and P2 defined on left posterior-lateral portion of the neck 110. Together, the sections P8, P1, and P2 form a left posterior-lateral neck toning treatment grid.

The beam 122 (see Figure 3) may be applied to each of the sections P1-P8 to thereby apply circumferential treatment (360 degrees) around the neck 110. Such circumferential treatment tones neck muscles (e.g., the platysma muscle and posterior neck muscles) and connective tissue associated with (or attached to) these muscles together as a unit. This toning provides a circumferential reduction, which clinically translates into a more toned, lifted, and/or "pedestal" neck with reduced or eliminated forward sag.

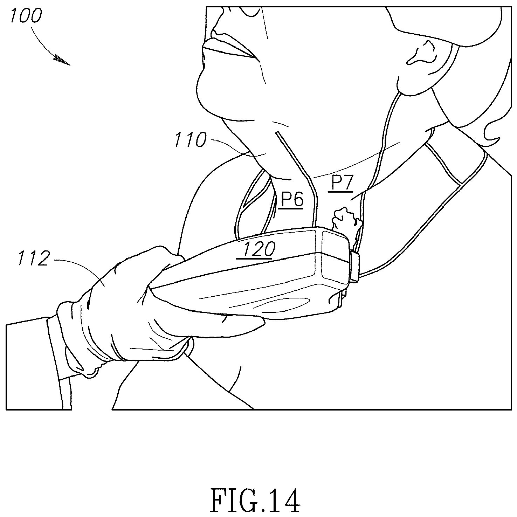

Referring to Figure 3, a layer of gel 150 (e.g., refrigerated or cooled water soluble ultrasound gel) may be applied to the exterior surface 13 (see Figure 3) of the skin 130 in the target region 144 before the target region 144 is treated with the device 120. Figure 13 illustrates the healthcare provider 112 applying the gel 150 to the anterior neck toning treatment grid, and Figure 14 illustrates the healthcare provider 112 using the device 120 to apply the beam 122 (see Figure 3) to the anterior neck toning treatment grid through the gel 150.

As will be readily appreciated by those of ordinary skill in the art, any number of gels may be used in connection with applying the beam 122 to the outer surface of the target region 144. After the gel 150 is applied, the treatment window 140 may be placed against the outer surface of the target region 144 and used to deliver the beam 122 to the outer surface of the target region 144 through the gel 150. Then, the gel 150 may be wiped away. The process of applying the gel 150, using the device 120, and wiping away the gel 150 may be repeated one or more times.

Optionally, referring to Figure 3, a layer of a composition 152 may be applied to the exterior surface 13 (see Figure 3) of the skin 130 of the target region 144 before the target region 144 is treated with the device 120. For example, the composition 152 may be applied to the outer surface of the target region 144 first. Then, the gel 150 may be applied over the composition 152. In such embodiments, the composition 152 lies substantially between the outer surface of the target region 144 and the gel 150. After the composition 152 and the gel 150 are applied to the outer surface of the target region 144, the treatment window 140 may be placed against the outer surface of the target region 144 and used to deliver beam 122 to the outer surface of the target region 144 through the composition 152 and the gel 150. Then, the composition 152 and the gel 150 may be wiped away. The process of applying the composition 152, applying the gel 150, using the device 120, and wiping away the composition 152 and the gel 150 may be repeated one or more times.

The composition 152 may be inert and may not be absorbable by the skin. By way of a non-limiting example, the composition 152 may be in the form of a powder that includes one or more of the following ingredients in varying amounts: micronized zinc oxide, micronized titanium dioxide, pigmenting titanium dioxide, iron oxide, oat, rice, mica, silicone powder, marine algae and/or talc. As used herein, the term "micronized" describes a relatively small particle size (especially with regard to the particle size of like compounds used in traditional sunscreen products), which may be, for instance, in the range of about 30 µm to about 50 µm, and in certain embodiments, of about 40 µm. As used herein, "pigmenting titanium dioxide" is a form of titanium dioxide with a relatively large particle size, which may be, for instance, at least 850 µm, at least 900 µm, at least 950 µm and/or up to about 1,000 µm. The composition 152 may be formulated to include particles of varying size so as to reflect, refract, and/or scatter light exposed to the composition 152 in a generally predetermined manner. The composition 152 may include one or more compositions that may be obtained from Colorescience (Dana Point, Calif.). For example, the composition 152 may include Sunforgettable Mineral Powder Sun Protection SPF 50 and/or Pressed Illuminating Pearl Powder.

The composition 152 may also include a quantity of salicylic acid, which may itself be in powder form. Such salicylic acid powder includes a predetermined concentration of powered salicylic acid, such as 0.01%, 0.05%, 0.1%, 0.5%, 1.0%, 1.5%, 2.0%, 2.5%, or 3.0%. In an embodiment of the present invention, the salicylic acid powder includes 1% powdered salicylic acid.

Referring to Figure 3, preparing the outer surface of the target region 144 with the composition 152 and the gel 150, as described above, augments the skin tightening effect, while advantageously cooling the exterior surface 13 of the skin 130 of the target region 144 through the inherent properties of minerals and mineral pigments, which are known to have a surface cooling effect. The device 120 delivers the beam 122 (e.g., infrared light) that heats the dermal layer of the skin 130. Sustained heating of the dermal layer of the skin 130 (see Figure 2) over several seconds (e.g., up to six seconds) contracts the collagen and elastin components of the dermis and/or causes long-term stimulation of collagen and elastin remodeling through fibroblast activity, resulting in tightening of the skin 130, reduction of the skin 130, and/or improved skin tone. Additionally, the connective tissue 20 and/or the fascia 22 may be reduced, tightened, and/or toned..

By way of a non-limiting example, the composition 152 (e.g., including micronized zinc oxide, micronized titanium dioxide, mica, and 1% salicylic acid powder) may be applied to the outer surface of the target region 144. Then, the gel 150 may applied atop the composition 152. Next, the treatment window 140 is placed firmly onto the skin 130 in the target region 144, and the beam 122 is applied to the target region 144. Then, the beam 122 is discontinued and the treatment window 140 is moved away from the target region 144. Within the span of several seconds, the skin 130 has been pre-cooled (by the gel 150), and heated with the beam 122. Optionally, the skin 130 may be post-cooled (e.g., by a new layer of the gel 150). This process (referred as a "pulse") may be repeated for different target regions in the treatment area 124 with minimal overlap until the entire treatment area 124 has been treated. As used herein, a series of pulses required to substantially cover the treatment area 124 is referred to as a "pass." Upon completion of a first pass, additional passes may be performed until a desirable number of passes have been performed. The desirable number of passes may be readily determined in each instance by a skilled artisan, based on a variety of factors, such as, but not limited to, the condition of the treatment area before treatment and the response of the treatment area to one or more passes.

Once the desired number of passes are completed, treatment of the treatment area 124 is completed. Treatment of the treatment area 124 may result in some immediate reduction in skin and ongoing reduction of skin over several weeks thereafter.

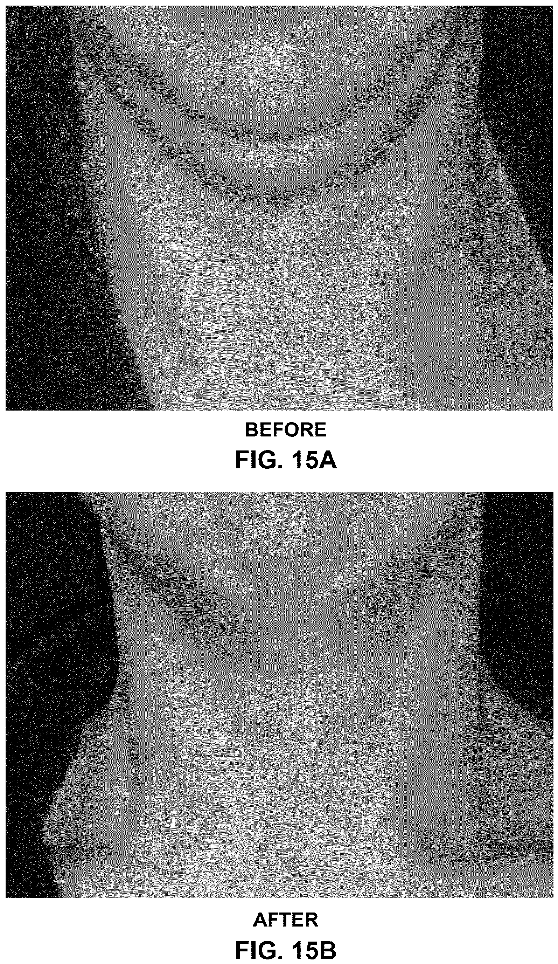

Figures 15A-15B are photographs depicting exemplary results achieved by a patient. Figure 15A depicts an anterior portion of the patient's neck before treatment, and Figure 15B depicts the anterior portion of the patient's neck after treatment. As may be viewed in Figures 15A-15B, the treatment improved the tone of the patient's neck.

The methods described above are non-invasive, require no anesthesia, and require no recovery time. These methods may be used tone skin having mild, moderate, or severe laxity, wrinkling, and/or sagging. Additionally, these methods may be useful for women and men regardless of their age or skin color.

The foregoing described embodiments depict different components contained within, or connected with, different other components. It is to be understood that such depicted architectures are merely exemplary, and that in fact many other architectures can be implemented which achieve the same functionality. In a conceptual sense, any arrangement of components to achieve the same functionality is effectively "associated" such that the desired functionality is achieved. Hence, any two components herein combined to achieve a particular functionality can be seen as "associated with" each other such that the desired functionality is achieved, irrespective of architectures or intermedial components. Likewise, any two components so associated can also be viewed as being "operably connected," or "operably coupled," to each other to achieve the desired functionality.

While particular embodiments of the present invention have been shown and described, it will be obvious to those skilled in the art that, based upon the teachings herein, changes and modifications may be made without departing from this invention and its broader aspects and, therefore, the appended claims are to encompass within their scope all such changes and modifications as are within the true spirit and scope of this invention. Furthermore, it is to be understood that the invention is solely defined by the appended claims. It will be understood by those within the art that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to inventions containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations. In addition, even if a specific number of an introduced claim recitation is explicitly recited, those skilled in the art will recognize that such recitation should typically be interpreted to mean *at least* the recited number (e.g., the bare recitation of "two recitations," without other modifiers, typically means *at least* two recitations, or *two or more* recitations).

Accordingly, the invention is not limited except as by the appended claims.

## Claims

1. A method of toning an anterior portion of skin on a human patient's neck, the neck comprising a posterior portion of skin opposite the anterior portion, the method comprising:
applying a beam comprising at least one of light waves, ultrasound waves, and low fluence radiofrequency waves to an exterior surface of the posterior portion of the skin of the neck, the beam being configured to tone the posterior portion of the skin causing the posterior portion of the skin to pull on and tighten the anterior portion of the skin.

2. The method of claim 1, applying a layer of gel to the external surface of the posterior portion of the skin of the neck before applying the beam, and applying the beam to the exterior surface of the posterior portion through the layer of gel.

3. The method of claim 1, applying the beam to at least one portion of the skin of the neck other than the posterior portion.

4. The method of claim 1, wherein the beam comprises only infrared light.

5. The method of claim 1, wherein the beam comprises only broadband light having wavelengths of approximately 700 nm to approximately 1300 nm.

6. The method of claim 1, wherein the beam is emitted by a micro pinpoint ultrasound device.

7. The method of claim 1, wherein the beam comprises low fluence radiofrequency.

8. A method of toning skin on a human patient's neck, the skin being divisible circumferentially around the neck into a plurality of sections, each section extending only partway around the neck, the method comprising:
applying a beam comprising at least one of light waves, ultrasound waves, and low fluence radiofrequency waves to each of the plurality of sections separately, application of the beam toning the skin on the human patient's neck.

9. The method of claim 8, applying gel to each of the plurality of sections before the beam is applied thereto, wherein the beam is applied to each of the plurality of sections through the gel.

10. The method of claim 8, wherein the beam comprises at least one of infrared light and broadband light, wherein the broadband light has wavelengths of approximately 700 nm to approximately 1300 nm.

11. The method of claim 8, wherein the beam is emitted by a micro pinpoint ultrasound device.

12. A method of toning skin on a human patient's neck, the method comprising applying a beam comprising at least one of light waves, ultrasound waves, and low fluence radiofrequency waves to a portion of the skin extending circumferentially completely around the neck, the beam being configured to tone the portion of the skin.

13. The method of claim 12, applying gel to the portion of the skin before the beam is applied thereto, wherein the beam is applied to the portion of the skin through the gel.

14. The method of claim 12, wherein the beam comprises at least one of infrared light and broadband light, wherein the broadband light has wavelengths of approximately 700 nm to approximately 1300 nm.

15. The method of claim 12, wherein the beam is emitted by a micro pinpoint ultrasound device.
